(19) Europäisches Patentamt
European Patent Office
Office européen des brevets

(11) **EP 3 141 887 A1**

(12) **EUROPÄISCHE PATENTANMELDUNG**

(43) Veröffentlichungstag:
**15.03.2017 Patentblatt 2017/11**

(21) Anmeldenummer: **15184462.8**

(22) Anmeldetag: **09.09.2015**

(51) Int Cl.:
*G01N 21/15* (2006.01)    *G01N 21/27* (2006.01)
*G01N 21/31* (2006.01)    *G01N 33/49* (2006.01)
*G01N 35/00* (2006.01)    *G01N 21/17* (2006.01)
*G01J 3/42* (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA**

(71) Anmelder: **Siemens Healthcare Diagnostics Products GmbH**
**35041 Marburg (DE)**

(72) Erfinder: **MELLER, Paul**
**61273 WEHRHEIM (DE)**

(54) **VERFAHREN ZUM BETREIBEN EINES PHOTOMETERS**

(57) Ein Verfahren zum Betreiben eines Photometers (8) in einem automatischen Analysegerät (1), bei dem eine Probenmessung erfolgt, indem eine Körperflüssigkeit in eine Durchflussmesszelle (24) eingeführt wird, Licht aus einer Lichtquelle (16) durch die Durchflussmesszelle (24) auf ein Lichtmesselement (20) gestrahlt wird und die optische Dichte bestimmt wird, soll einen besonders ressourcenschonenden und gleichzeitig fehlerarmen Betrieb des Photometers (8) in einem automatischen Analysegerät (1) erlauben. Dazu erfolgt vor der Probenmessung eine Referenzmessung, indem ein Referenzmedium in die Durchflussmesszelle (24) eingeführt wird, die optische Dichte bestimmt wird und bei Überschreiten eines ersten vorgegebenen Schwellwerts für die optische Dichte ein der Durchflussmesszelle (24) zugeordnetes Austauschsignal erzeugt wird.

FIG 2

EP 3 141 887 A1

**Beschreibung**

Die Erfindung liegt auf dem Gebiet der automatischen Analysegeräte und betrifft ein Verfahren zum Betreiben eines Photometers in einem automatischen Analysegerät, bei dem eine Probenmessung erfolgt, indem eine Körperflüssigkeit in eine Durchflussmesszelle eingeführt wird, Licht aus einer Lichtquelle durch die Durchflussmesszelle auf ein Lichtmesselement gestrahlt wird und die optische Dichte bestimmt wird.

Heutige Analysegeräte, wie sie routinemäßig in der Analytik, der Forensik, der Mikrobiologie und der klinischen Diagnostik Verwendung finden, sind in der Lage, eine Vielzahl von Nachweisreaktionen und Analysen mit einer Vielzahl von Proben durchzuführen. Um eine Vielzahl von Untersuchungen automatisiert durchführen zu können, sind diverse automatisch arbeitende Vorrichtungen zum räumlichen Transfer von Messzellen, Reaktionsbehältern und Reagenzflüssigkeitsbehältern, wie z. B. Transferarme mit Greiffunktion, Transportbänder oder drehbare Transporträder, sowie Vorrichtungen zum Transfer von Flüssigkeiten, wie z.B. Pipettiervorrichtungen, in einem Gerätegehäuse untergebracht. Die Geräte umfassen eine zentrale Steuereinheit, die mittels entsprechender Software dazu in der Lage ist, die Arbeitsschritte für die gewünschten Analysen weitgehend selbstständig zu planen und abzuarbeiten.

Viele der in derartigen automatisiert arbeitenden Analysegeräten verwendeten Analyseverfahren beruhen auf optischen Methoden. Besonders verbreitet sind Messsysteme, die auf photometrischen (z.B. turbidimetrischen, nephelometrischen, fluorometrischen oder luminometrischen) oder radiometrischen Messprinzipien beruhen. Diese Verfahren ermöglichen den qualitativen und quantitativen Nachweis von Analyten in flüssigen Proben, ohne zusätzliche Trennschritte vorsehen zu müssen. Die Bestimmung klinisch relevanter Parameter, wie zum Beispiel der Konzentration oder der Aktivität eines Analyten, erfolgt vielfach, indem ein Aliquot einer Körperflüssigkeit eines Patienten gleichzeitig oder nacheinander mit einem oder mehreren Testreagenzien in einem Reaktionsgefäß zu einem Reaktionsansatz vermischt wird, wodurch eine biochemische Reaktion in Gang gesetzt wird, die eine messbare Veränderung einer optischen Eigenschaft des Reaktionsansatzes bewirkt.

Das Messergebnis wird von dem Messsystem wiederum in eine Speichereinheit weitergeleitet und ausgewertet. Anschließend liefert das Analysegerät einem Benutzer über ein Ausgabemedium, wie z.B. einen Monitor, einen Drucker oder eine Netzwerkverbindung probenspezifische Messwerte.

Vor der eigentlichen Bestimmung eines Analyten in einer Probe wird im Zuge der automatisierten Probenanalytik zunehmend eine präanalytische Voruntersuchung der Probe auf probenintrinsische Störsubstanzen durchgeführt. Körperflüssigkeitsproben können in Einzelfällen abnormal hohe Konzentrationen einer oder mehrerer intrinsischer, also körpereigener Substanzen enthalten, die sich bei Überschreitung einer tolerablen Konzentration in photometrischen Detektionsverfahren als störend erweisen und sich zu einem systematischen Fehler auswirken können. Probleme bereiten bekanntermaßen hämolytische, ikterische und/oder lipämische Serum- oder Plasmaproben, die über abnormal hohe Hämoglobin-, Bilirubin- und/oder Lipid-Konzentrationen verfügen. Abnormal hohe Konzentrationen dieser interferierenden Substanzen können durch einen pathologischen Zustand des Patienten oder aber durch eine unsachgemäße Probengewinnung oder -lagerung verursacht werden. Werden Testansätze solcher Primärproben einem photometrischen Verfahren unterworfen, das der Bestimmung eines analytischen, diagnostisch relevanten Parameters dient, besteht die Gefahr einer Fehlbestimmung, die gegebenenfalls eine Fehldiagnose und schlimmstenfalls eine Fehlbehandlung des Patienten zur Folge haben kann. Die präanalytische Identifikation hämolytischer, ikterischer sowie lipämischer Proben ist also zur Vermeidung von fehlerhaften Analyseergebnissen von besonderer Wichtigkeit.

Neuere automatische Analysegeräte umfassen Systeme, die die genannten Interferenzen automatisiert vor Beginn der eigentlichen Analyse ermitteln können. Hierzu kommen unter anderem geeignete Photometer zur Anwendung, in denen zunächst eine kleine Menge der Patientenprobe in eine Durchflussmesszelle eingebracht wird und Licht geeigneter Wellenlänge aus einer Lichtquelle durch die Durchflussmesszelle auf ein Lichtmesselement gestrahlt und die optische Dichte bestimmt wird. Ein derartiges Photometersystem ist beispielsweise in der WO 2012/151358 A2 beschrieben.

Die Verwendung einer Durchflussmesszelle bietet zwar den Vorteil, dass eine Vielzahl von Proben in kurzer Zeit untersucht werden kann, allerdings weisen derartige Durchflussmesszellen nur eine begrenzte Haltbarkeit auf, da aufgrund ihrer geringen Größe (Volumen ca. 20-50 μl) und der wiederholten Kontamination mit Bestandteilen von Patientenproben (einige hundert Proben pro Tag) und dadurch an den Wänden der Durchflussmesszelle anhaftenden Probenbestandteilen eine Trübung einsetzt, die die Messauflösung einschränkt und Messergebnisse verfälscht. Daher wird die Messzelle in der Regel als auswechselbare Komponente ausgeführt.

Hierbei ergibt sich jedoch das Problem, den geeigneten Zeitpunkt für eine Auswechselung der Durchflussmesszelle zu ermitteln. Eine zu späte Auswechselung der Durchflussmesszelle führt unter Umständen zu Messfehlern, während ein zu häufiges Auswechseln Stillstandzeiten des Analysegeräts und einen hohen Ressourcenverbrauch zur Folge hat.

Es ist daher Aufgabe der Erfindung, ein Verfahren anzugeben, welches einen besonders ressourcenschonenden und gleichzeitig fehlerarmen Betrieb einer photometrischen Vorrichtung mit einer auswechselbaren Durchflussmesszelle in einem automatischen Analysegerät erlaubt.

Diese Aufgabe wird erfindungsgemäß durch eine kontinuierliche Überwachung der Qualität der Messzelle gelöst, und zwar indem vor der Probenmessung in der Durchflussmesszelle eine Referenzmessung erfolgt, in der Weise, dass ein Referenzmedium in die Durchflussmesszelle eingeführt wird, die optische Dichte bestimmt wird und bei Überschreiten eines ersten vorgegebenen Schwellwerts für die optische Dichte ein der Durchflussmesszelle zugeordnetes Austauschsignal erzeugt wird.

Es wurde gefunden, dass die Zuverlässigkeit der Probenmessung dadurch gewährleistet werden kann, dass vor der Messung der Probe in der Durchflussmesszelle eine Überprüfung der Trübung der Durchflussmesszelle erfolgt. Dies erfolgt in Form einer Referenzmessung, bei der vor dem Einführen der eigentlichen zu messenden Probenflüssigkeit eine Referenzflüssigkeit mit bekannter, immer gleicher optischer Dichte eingeführt wird, z.B. Wasser, und anhand der die Trübung der Wandung der Durchflussmesszelle ermittelt werden kann. Wird die Trübung zu stark, was durch Überschreiten eines vorgegebenen Schwellwerts für die optische Dichte bei der Messung der Referenzflüssigkeit erkannt werden kann, wird ein Austauschsignal an einen Benutzer ausgegeben. Dieser kann dann gezielt einen Austausch der Durchflussmesszelle durchführen.

Vorteilhafterweise erfolgt während mehrerer aufeinanderfolgender Probenmessungen zyklisch wiederkehrend eine Referenzmessung. Das bedeutet, dass zwischen mehreren, in vergleichsweise hoher Zahl erfolgenden Probenmessungen in regelmäßigem Abstand immer wieder eine Referenzmessung zwischen zwei Probenmessungen erfolgt. Der Abstand kann dabei als fester Zeitraum gegeben sein, d.h. dass z.B. alle X Minuten oder alle Y Stunden eine Referenzmessung durchgeführt wird, oder aber in Anzahlen von Probenmessungen bemessen sein, d.h. dass stets nach einer bestimmten Anzahl von Probenmessungen eine Referenzmessung vor der nächsten Probenmessung durchgeführt wird. Hierdurch wird gewährleistet, dass stets der aktuelle Verschleißzustand der Durchflussmesszelle bekannt ist.

Besonders vorteilhaft erfolgt vor jeder Probenmessung eine Referenzmessung. Hierdurch wird für jede einzelne Probenmessung vorab die Qualität der Durchflussmesszelle ermittelt, so dass für jede Probenmessung das Fehlerrisiko minimiert wird.

In weiterer vorteilhafter Ausgestaltung des Verfahrens wird ein zweiter Schwellwert für die optische Dichte der Referenzmessung vorgegeben, welcher niedriger ist als der erste Schwellwert, der das Austauschsignal erzeugt. Wird während der Referenzmessung eine optische Dichte bestimmt, die zwar den ersten Schwellwert noch nicht überschreitet, aber die diesen zweiten vorgegebenen Schwellwert überschreitet, wird ein der Durchflussmesszelle zugeordnetes Vorwarnsignal erzeugt. Hierdurch kann bereits vor dem notwendigen Austausch der Durchflussmesszelle dem Benutzer ein bevorstehender notwendiger Austausch angekündigt werden, so dass eine entsprechende Ausfallzeit für den Austausch planbar wird.

Als Reaktion auf das Erzeugen des Vorwarnsignals kann vorteilhafterweise zudem eine automatisierte Intensivreinigung der Durchflussmesszelle erfolgen. Unter einer Intensivreinigung wird hierbei eine Reinigung verstanden, die über das übliche Spülverfahren der Durchflussmesszelle zwischen der Befüllung mit einzelnen Probenflüssigkeiten hinausgeht und eine dementsprechend stärkere Reinigungsleistung aufweist, z.B. durch Verwendung einer speziellen Reinigungsflüssigkeit, längere Einwirkzeiten oder durch eine mechanische Reinigung. Hierdurch kann die Nutzungsdauer der Durchflussmesszelle verlängert werden.

Das Überschreiten eines der oben beschriebenen vorgegebenen Schwellwerte kann auf verschiedene Arten ermittelt werden, z.B. anhand der aktuell ermittelten optischen Dichte in der Referenzmessung. In besonders vorteilhafter Ausgestaltung des Verfahrens wird das Überschreiten jedoch anhand eines gleitenden Mittelwerts für die optische Dichte bei einer Mehrzahl von Referenzmessungen ermittelt. Hierdurch wird das Auslösen eines Austausch- oder Vorwarnsignals aufgrund einzelner überhöhter Messwerte vermieden. So kann vorteilhaft z.B. das Mittel der optischen Dichten der jeweils letzten n (n ≥ 10) Referenzmessungen als Vergleichswert herangezogen werden.

Alternativ kann die optische Dichte einer Referenzmessung als relative optische Dichte im Vergleich zu einer Referenzmessung mit einer neu eingesetzten Durchflussmesszelle ermittelt werden. Mit anderen Worten: Beim Einsetzen einer neuen Durchflussmesszelle wird eine allererste Referenzmessung durchgeführt und so ein Anfangswert für die optische Dichte ermittelt, zu dem die zu einem späteren Zeitpunkt jeweils aktuell in einer Referenzmessung gemessene optische Dichte in Bezug gesetzt wird.

Vorteilhafterweise wird die relative optische Dichte dabei als Zehnerlogarithmus aus dem Quotienten des von dem Lichtmesselement gemessenen Lichtsignals der allerersten Referenzmessung mit der neu eingesetzten Durchflussmesszelle und des von dem Lichtmesselement gemessenen Lichtsignals der aktuellen Referenzmessung definiert. Dies entspricht der üblichen Definition der optischen Dichte, wobei die Intensität der einfallenden Strahlung durch die Intensität der austretenden Strahlung bei der allerersten Referenzmessung einer neu eingesetzten Durchflussmesszelle ersetzt wurde. Die Definition stellt einen einfach zu handhabenden Wert für den im Rahmen des Verfahrens notwendigen Vergleich dar.

Um die Erkennung für die Austauschbedingung der Küvette zu gewährleisten, sollte dabei ein Schwellwert definiert werden, der so gewählt wird, dass das Auflösungsvermögen der Messmethode nicht eingeschränkt wird und die benötigte Auflösung für die zu messenden Proben nicht eingeschränkt wird. Der erste Schwellwert, d.h. der Schwellwert, dessen Überschreiten als Reaktion die Erzeugung eines Austauschsignals zur Folge hat, liegt vorteilhafterweise zwischen 0,4 und 0,8, noch vorteilhafter zwischen 0,5 und 0,7.

Der zweite Schwellwert, d.h. der Schwellwert, dessen Überschreiten als Reaktion die Erzeugung eines Vorwarnsignals und gegebenenfalls eine Intensivreinigung zur Folge hat, liegt vorteilhafterweise zwischen 0,2 und 0,6, noch vorteilhafter zwischen 0,3 und 0,5.

Ein weiterer Gegenstand der vorliegenden Erfindung ist ein automatisches Analysegerät mit einem Photometer, wobei das Photometer eine austauschbare Durchflussmesszelle, mindestens eine Lichtquelle und mindestens ein Lichtmesselement umfasst, die derart angeordnet sind, dass Licht aus der Lichtquelle durch die Durchflussmesszelle auf das Lichtmesselement gestrahlt werden kann. Das automatische Analysegerät umfasst ferner eine Steuerung, vorzugsweise mit entsprechender Software, die das Analysegerät zum Ausführen des erfindungsgemäßen Verfahrens veranlasst. Insbesondere ist die Steuerung so konfiguriert, dass sie ein Verfahren mit den folgenden Schritten steuert:

- Einführen eines Referenzmediums in die Durchflussmesszelle und Bestimmen der optischen Dichte,
- Vergleichen der bestimmten optischen Dichte mit einem ersten vorgegebenen Schwellwert für die optische Dichte und
- Erzeugen eines der Durchflussmesszelle zugeordneten Austauschsignals, wenn die bestimmte optische Dichte den ersten vorgegebenen Schwellwert für die optische Dichte überschreitet.

Lichtquelle und Lichtmesselement des Photometers sind vorteilhafterweise für Wellenlängen zwischen 340 nm und 380 nm und/oder zwischen 405 nm und 425 nm und/oder zwischen 360 nm und 480 nm und/oder zwischen 600 nm und 800 nm ausgebildet. Hierfür kann die Lichtquelle z.B. entsprechende Filter umfassen, oder das Photometer kann mehrere Lichtquellen, z.B. Leuchtdioden, umfassen. Diese Wellenlängen sind für die Erkennung von Hämolyse (H), Ikterus (I) und Lipämie (L) besonders geeignet.

Vorzugsweise ist die Steuerung des automatischen Analysegeräts ferner so konfiguriert, dass das von der Steuerung gesteuerte Verfahren zusätzlich die folgenden Schritte umfasst:

- Vergleichen der bestimmten optischen Dichte mit einem zweiten vorgegebenen Schwellwert für die optische Dichte und
- Erzeugen eines der Durchflussmesszelle zugeordneten Vorwarnsignals, wenn die bestimmte optische Dichte den zweiten vorgegebenen Schwellwert für die optische Dichte überschreitet.

Ein besonders bevorzugtes automatisches Analysegerät weist ferner eine optische Anzeige auf, beispielsweise einen Bildschirm. Vorzugsweise ist die vorbeschriebene Steuerung dann so konfiguriert, dass das von der Steuerung gesteuerte Verfahren zusätzlich die folgenden Schritte umfasst:

- Anzeigen des der Durchflussmesszelle zugeordneten Austauschsignals und/oder des der Durchflussmesszelle zugeordneten Vorwarnsignals in Form einer Textnachricht oder in Form eines Piktogramms.

Alternativ oder zusätzlich kann das automatische Analysegerät auch einen Lautsprecher aufweisen, wobei die Steuerung dann so konfiguriert ist, dass das der Durchflussmesszelle zugeordnete Austauschsignal und/oder das der Durchflussmesszelle zugeordnete Vorwarnsignal in Form eines akustischen Warnsignals abgegeben wird.

Dies stellt sicher, dass ein Anwender über den Status der Durchflussmesszelle informiert wird und rechtzeitig einen Austausch der Durchflussmesszelle vorbereiten oder durchführen kann.

Die mit der Erfindung erzielten Vorteile bestehen insbesondere darin, dass durch die regelmäßige Referenzmessung einer austauschbaren Durchflussmesszelle in einem Photometer eine Verschmutzung frühzeitig erkannt wird und ein Austausch planbar wird. Dadurch werden Fehlmessungen durch eine Verschmutzung konsequent vermieden.

Ein Ausführungsbeispiel der Erfindung wird anhand einer Zeichnung näher erläutert. Darin zeigen:

FIG 1      eine schematische Darstellung eines automatischen Analysegeräts,

FIG 2      eine schematische Darstellung eines HIL-Photometers, und

FIG 3 und FIG 4      eine schematische Darstellung der Verfahrensschritte eines Verfahrens zur Ermittlung der Qualität und Eignung der Durchflussmesszelle des HIL-Photometers.

Gleiche Teile sind in allen Figuren mit denselben Bezugszeichen versehen.

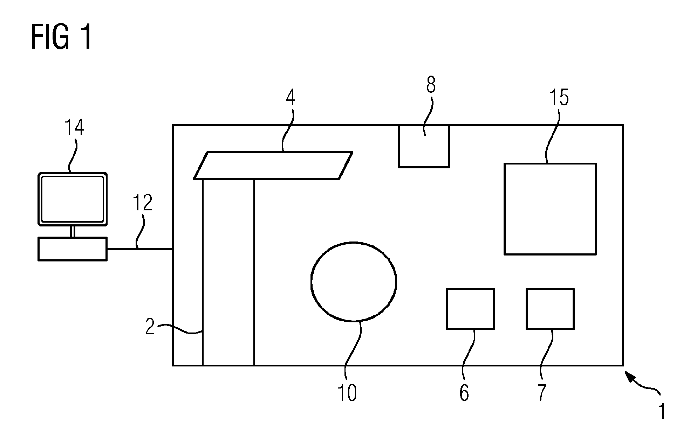

FIG 1 zeigt eine schematische Darstellung eines automatischen Analysegeräts 1 mit einigen darin enthaltenen Bauteilen. Hierbei werden nur die wichtigsten Bauteile stark vereinfacht dargestellt, um die grundsätzliche Funktion des automatischen Analysegeräts 1 zu erläutern, ohne hierbei detailliert die einzelnen Teile jedes Bauteils darzustellen.

Das automatische Analysegerät 1 ist dafür ausgebildet, vollautomatisch verschiedenste Analysen von Blutplasma oder anderen Körperflüssigkeiten durchzuführen, ohne dass hierfür Aktivitäten eines Benutzers notwendig wären. Notwendige Eingriffe eines Benutzers beschränken sich vielmehr auf Wartung oder Reparatur und Nachfüllarbeiten, wenn z.B. Verbrauchsmaterial, wie Küvetten nachgefüllt oder Reagenzflüssigkeitsbehälter ausgetauscht werden müssen.

Die Probengefäße mit Patientenproben werden dem automatischen Analysegerät 1 auf nicht näher dargestellten Schlitten in einer Zuführungsschiene 2 zugeführt. Informationen hinsichtlich der pro Probe durchzuführenden Analysen können hierbei beispielsweise mittels auf den Probengefäßen angebrachten Strichcodes übergeben werden, die im automatischen Analysegerät 1 ausgelesen werden. Aus den Probengefäßen werden in einer Pipettiereinrichtung 4 Aliquots mittels einer nicht näher dargestellten Pipettiernadel entnommen.

Die Aliquots werden ebenfalls nicht näher dargestellten Küvetten zugeführt, die in Aufnahmepositionen einer drehbaren, auf 37 °C temperierten Inkubationseinrichtung 10 angeordnet sind. In dem auf ca. 8-10 °C gekühlten Reagenzgefäßvorratsbehälter 15 werden Reagenzgefäße mit verschiedenen Reagenzflüssigkeiten aufbewahrt. Reagenzflüssigkeit wird mittels der Pipettiernadel einer zweiten Pipettiervorrichtung (nicht dargestellt) aus einem Reagenzgefäß entnommen und zur Bereitstellung eines Testansatzes in eine Küvette, die bereits ein Probenaliquot enthält, abgegeben. Die Küvette mit dem Testansatz wird von einem nicht dargestellten Transferarm mit einem Greifer von der Inkubationseinrichtung 10 in eine photometrische Messeinheit 6,7 transportiert, wo die Extinktion des Testansatzes gemessen wird. Die Extinktionsmessung des Testansatzes dient der quantitativen Bestimmung eines Analyten, beispielsweise der Aktivitätsbestimmung eines Blutgerinnungsfaktors.

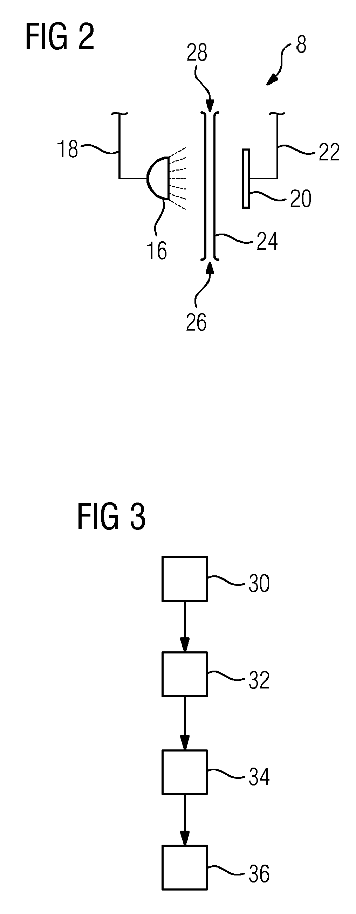

Für die präanalytische Analyse der unverdünnten Probenflüssigkeiten ist ein weiteres Photometer 8 mit einer auswechselbaren Durchflussmesszelle vorgesehen, welches in Bezug auf FIG 2, FIG 3 und FIG 4 noch näher erläutert wird. Vor der eigentlichen Bestimmung eines Analyten in einer mit Testreagenzien vermischten Probe in den photometrischen Messeinheiten 6,7 wird ein Aliquot jeder Patientenprobe im Photometer 8 auf Hämolyse, Ikterus und Lipämie untersucht. Das Photometer 8 ist schematisch in FIG 2 dargestellt.

Der gesamte Prozess wird von einer Steuereinrichtung, wie z.B. einem über eine Datenleitung 12 angeschlossenen Rechner 14 gesteuert, unterstützt durch eine Vielzahl weiterer, nicht näher dargestellter elektronischer Schaltungen und Mikroprozessoren innerhalb des automatischen Analysegeräts 1 und seiner Bauteile.

Das Photometer 8 gemäß der FIG 2 umfasst eine Lichtquelle 16, z.B. eine Anordnung von Leuchtdioden, die ggf. mit Filtern versehen sein können, die von einer Stromleitung 18 mit Strom versorgt wird und so ausgerichtet ist, dass sie Licht auf ein ebenfalls entsprechend ausgerichtetes Lichtmesselement 20 strahlt, dessen Signal über eine Signalleitung 22 an eine nicht näher dargestellte Steuerungseinrichtung des automatischen Analysegeräts 1 übertragen wird. Das Lichtmesselement 20 kann hierbei beispielsweise als Photodiode ausgestaltet sein. Das Licht tritt auf dem Weg von der Lichtquelle 16 zum Lichtmesselement 20 durch eine Durchflussmesszelle 24, an deren Eintritt 28 ein Aliquot einer Patientenprobe oder zur Referenzmessung Wasser eingeführt wird, welches durch den Austritt 26 nach erfolgter Probenmessung wieder abgeleitet wird.

Lichtquelle 16 und Lichtmesselement 20 sind für eine gezielte Messung der optischen Dichte bei Licht der Wellenlängen von 360 nm, 415 nm, 470 nm und 630 nm ausgebildet, entweder durch entsprechende Ausbildung von Leuchtdioden entsprechender Wellenlängen, die Nutzung von Filtern oder entsprechender Eigenschaften des Lichtmesselements 20. Das Photometer 8 eignet sich damit zur Ermittlung von Hämolyse, Ikterus und Lipämie in einer Patientenprobe und wird daher auch als HIL-Photometer bezeichnet. Die genaue Auswertung der Probenmessung im Rahmen der HIL-Präanalyse ist dem Fachmann bekannt und wird hier nicht weiter detailliert beschrieben.

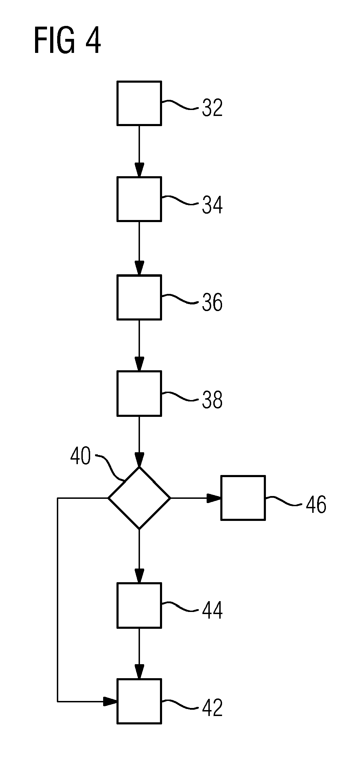

Da die Durchflussmesszelle 24 ein Verschleißteil darstellt, da sie nach und nach im Laufe der Probenmessungen infolge der Adsorption von Probenbestandteilen mehr und mehr eintrübt, ist sie als auswechselbares Bauteil ausgebildet. Um den Austausch in jedem Fall rechtzeitig durchzuführen und dem Benutzer des automatischen Analysegeräts 1 eine Planbarkeit des Austausches zu ermöglichen, wird nach dem in FIG 3 und FIG 4 beschriebenen Verfahren der Zustand der Durchflussmesszelle 24 zyklisch überprüft.

FIG 3 beschreibt zunächst die initialen Schritte nach dem Einbau einer neuen Durchflussmesszelle 24: In Schritt 30 wird eine neue Durchflussmesszelle 24 in das Photometer 8 eingesetzt. Die Durchflussmesszelle 24 wird dann in Schritt 32 mit Wasser als Referenzmedium gefüllt. Es erfolgt in Schritt 34 eine initiale, also allererste Referenzmessung, bei der - ebenso wie bei einer Probenmessung - die Lichtquelle 16 Licht durch die Durchflussmesszelle 24 auf das Lichtmesselement 20 strahlt. Der über die Signalleitung 22 bei dieser initialen Referenzmessung erfasste Signalwert wird im Folgenden mit s_0 bezeichnet. In Schritt 36 wird das Wasser wieder entfernt.

Die in FIG 4 dargestellten Schritte werden während des laufenden Betriebs des Photometers 8 vor jeder Probenmessung durchgeführt. Dies gilt zunächst für die Schritte 32 bis 36, d.h. vor jeder Probenmessung erfolgt eine Referenzmessung. Hierbei wird jeweils ein Signalwert über die Signalleitung 22 ermittelt, der mit s_j bezeichnet wird, wobei j die j-te Referenzmessung in Schritt 34 bezeichnet. Nach jeder erfolgten Referenzmessung erfolgt in Schritt 38 eine Berechnung der relativen optischen Dichte im Vergleich zum Signalwert s_0 mittels der Formel

$$OD(j) = \log(s\_0/s\_j).$$

Es wird sodann ein gleitender Mittelwert über die letzten zum Beispiel zehn (soweit vorhanden) relativen optischen Dichten gebildet. Hierfür werden die erfassten Werte jeweils in einem Speicher der Steuerungseinrichtung gespeichert. Dieser Mittelwert der relativen optischen Dichten der letzten zehn Messungen wird dann in Schritt 40 anhand vorgegebener Schwellwerte ausgewertet.

Liegt der Wert des Mittelwerts unterhalb von 0,4 OD(in anderen Ausführungsbeispielen können auch Werte zwischen 0,3 und 0,5 OD verwendet werden), so ist die Qualität der Durchflussmesszelle 24 noch ausreichend. Das Verfahren fährt in Schritt 42 mit der nächsten Probenmessung fort. Liegt der Wert des Mittelwerts oberhalb, jedoch noch unterhalb von 0,6 OD (in anderen Ausführungsbeispielen können auch Werte zwischen 0,5 und 0,7 OD verwendet werden), so wird in Schritt 44 ein Vorwarnsignal erzeugt. Dieses kann die Steuereinrichtung zu einer Benutzeranzeige veranlassen, die auf einen bevorstehenden Austausch hinweist, oder zu einer automatisierten Intensivreinigung der Durchflussmesszelle 24, z.B. mechanisch, durch spezielle Reinigungslösungen oder längere Einwirkzeiten derselben. In Schritt 42 fährt das Photometer 8 mit der nächsten Probenmessung fort.

Liegt jedoch in Schritt 40 der Mittelwert oberhalb des Schwellwertes von 0,6 OD, wird in Schritt 46 ein Austauschsignal erzeugt. Die Steuerungseinrichtung fordert den Benutzer auf, die Durchflussmesszelle 24 zu tauschen, da zuverlässige Messungen nicht mehr gewährleistet sind.

## BEZUGSZEICHENLISTE

| | |
|---|---|
| 1 | automatisches Analysegerät |
| 2 | Zuführungsschiene |
| 4 | Pipettiereinrichtung |
| 6, 7 | photometrische Messeinheit |
| 8 | Photometer mit Durchflussmesszelle |
| 10 | Inkubationseinrichtung |
| 12 | Datenleitung |
| 14 | Rechner |
| 15 | Reagenzgefäßvorratsbehälter |
| 16 | Lichtquelle |
| 18 | Stromleitung |
| 20 | Lichtmesselement |
| 22 | Signalleitung |
| 24 | Durchflussmesszelle |
| 26 | Austritt |
| 28 | Eintritt |
| 30-46 | Schritt |

## Patentansprüche

1. Verfahren zum Betreiben eines Photometers (8) in einem automatischen Analysegerät (1), bei dem eine Probenmessung erfolgt, indem eine Probenflüssigkeit in eine Durchflussmesszelle (24) eingeführt wird, Licht aus einer Lichtquelle (16) durch die Durchflussmesszelle (24) auf ein Lichtmesselement (20) gestrahlt wird und die optische Dichte bestimmt wird,

   **dadurch gekennzeichnet, dass** vor der Probenmessung eine Referenzmessung erfolgt, indem ein Referenzmedium in die Durchflussmesszelle (24) eingeführt wird, die optische Dichte bestimmt wird und mit einem ersten vorgegebenen Schwellwert für die optische Dichte verglichen wird, wobei bei Überschreiten des ersten vorgegebenen Schwellwerts für die optische Dichte ein der Durchflussmesszelle (24) zugeordnetes Austauschsignal erzeugt wird.

2. Verfahren nach Anspruch 1, bei dem mehrere Probenmessungen nacheinander erfolgen und wobei zyklisch wiederkehrend eine Referenzmessung zwischen zwei Probenmessungen erfolgt.

3. Verfahren nach Anspruch 1, bei dem vor jeder Probenmessung eine Referenzmessung erfolgt.

4. Verfahren nach einem der vorhergehenden Ansprüche, bei die optische Dichte der Referenzmessung zusätzlich mit einem zweiten vorgegebenen Schwellwert für die optische Dichte verglichen wird, welcher niedriger ist als der erste Schwellwert, wobei bei Überschreiten des zweiten vorgegebenen Schwellwerts für die optische Dichte ein der Durchflussmesszelle (24) zugeordnetes Vorwarnsignal erzeugt wird.

5. Verfahren nach Anspruch 4, bei dem als Reaktion auf das Erzeugen des Vorwarnsignals eine automatisierte Intensivreinigung der Durchflussmesszelle (24) erfolgt.

6. Verfahren nach einem der vorhergehenden Ansprüche, bei dem das Überschreiten eines der vorgegebenen Schwellwerte anhand eines gleitenden Mittelwerts für die optische Dichte bei einer Mehrzahl von Referenzmessungen ermittelt wird.

7. Verfahren nach einem der vorhergehenden Ansprüche, bei dem bei einer Referenzmessung die optische Dichte als relative optische Dichte im Vergleich zu der in der allerersten Referenzmessung für die Durchflussmesszelle (24) bestimmten optischen Dichte ermittelt wird.

8. Verfahren nach Anspruch 7, bei dem die relative optische Dichte als Zehnerlogarithmus aus dem Quotienten des von dem Lichtmesselement (20) gemessenen Lichtsignals der allerersten Referenzmessung mit einer Durchflussmesszelle (24) und des von dem Lichtmesselement (20) gemessenen Lichtsignals der aktuellen Referenzmessung definiert wird.

9. Verfahren nach Anspruch 8, bei dem der erste Schwellwert zwischen 0,4 und 0,8 liegt.

10. Verfahren nach einem der Ansprüche 4 und 8, bei dem der zweite Schwellwert zwischen 0,2 und 0,6 liegt.

11. Automatisches Analysegerät (1) mit einem Photometer (8), das Photometer (8) umfassend eine austauschbare Durchflussmesszelle (24), mindestens eine Lichtquelle (16) und mindestens ein Lichtmesselement (20), die derart angeordnet sind, dass Licht aus der Lichtquelle (16) durch die Durchflussmesszelle (24) auf das Lichtmesselement (20) gestrahlt werden kann, **dadurch gekennzeichnet, dass** das automatische Analysegerät (1) ferner eine Steuerung umfasst, die so konfiguriert ist, dass sie ein Verfahren mit den folgenden Schritten steuert:

- Einführen eines Referenzmediums in die Durchflussmesszelle (24) und Bestimmen der optischen Dichte,
- Vergleichen der bestimmten optischen Dichte mit einem ersten vorgegebenen Schwellwert für die optische Dichte und
- Erzeugen eines der Durchflussmesszelle (24) zugeordneten Austauschsignals, wenn die bestimmte optische Dichte den ersten vorgegebenen Schwellwert für die optische Dichte überschreitet.

12. Automatisches Analysegerät (1) gemäß Anspruch 11, wobei die Steuerung so konfiguriert ist, dass das von der Steuerung gesteuerte Verfahren zusätzlich die folgenden Schritte umfasst:

- Vergleichen der bestimmten optischen Dichte mit einem zweiten vorgegebenen Schwellwert für die optische Dichte und
- Erzeugen eines der Durchflussmesszelle (24) zugeordneten Vorwarnsignals, wenn die optische Dichte den zweiten vorgegebenen Schwellwert für die optische Dichte überschreitet.

13. Automatisches Analysegerät (1) gemäß einem der Ansprüche 11 und 12, wobei das Analysegerät ferner eine optische Anzeige aufweist und wobei die Steuerung so konfiguriert ist, dass das von der Steuerung gesteuerte Verfahren zusätzlich die folgenden Schritte umfasst:

- Anzeigen des der Durchflussmesszelle (24) zugeordneten Austauschsignals und/oder des der Durchflussmesszelle (24) zugeordneten Vorwarnsignals in Form einer Textnachricht oder in Form eines Piktogramms.

14. Automatisches Analysegerät (1) gemäß einem der Ansprüche 11 bis 13, bei dem die Lichtquelle (16) und das Lichtmesselement (20) des Photometers (8) für Wellenlängen zwischen 340 nm und 380 nm und/oder zwischen 405 nm und 425 nm und/oder zwischen 360 nm und 480 nm und/oder zwischen 600 nm und 800 nm ausgebildet sind.

FIG 1

FIG 2

28

8

18

22

20

16

24

26

FIG 3

30

32

34

36

FIG 4

Europäisches
Patentamt

European
Patent Office

Office européen
des brevets

**EUROPÄISCHER RECHERCHENBERICHT**

Nummer der Anmeldung

EP 15 18 4462

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2010/277727 A1 (SCHLAMINGER MICHAEL [AT]) 4. November 2010 (2010-11-04) | 1-13 | INV. G01N21/15 |
| Y | * Absatz [0001] - Absatz [0003]; Anspruch 1; Abbildung 1 * <br> * Absatz [0012] * <br> * Absatz [0017] - Absatz [0050] * <br> * Absatz [0070] * <br> ----- | 14 | G01N21/27 G01N21/31 G01N33/49 G01N35/00 <br><br> ADD. |
| Y | US 2008/297769 A1 (BAMBERG EBERHARD [US] ET AL) 4. Dezember 2008 (2008-12-04) * Absatz [0001] - Absatz [0002]; Abbildungen 3,18 * <br> * Absatz [0043] - Absatz [0045] * <br> * Absatz [0057] - Absatz [0071] * <br> ----- | 14 | G01N21/17 G01J3/42 |
| Y,D | WO 2012/151358 A2 (SIEMENS HEALTHCARE DIAGNOSTICS [US]; KRUFKA FRANK [US]) 8. November 2012 (2012-11-08) * Absatz [0005]; Anspruch 2; Abbildung 4 * <br> * Absatz [0020] * <br> * Absatz [0042] * <br> ----- | 14 | |

RECHERCHIERTE SACHGEBIETE (IPC)

G01N
G01J

Der vorliegende Recherchenbericht wurde für alle Patentansprüche erstellt

| Recherchenort | Abschlußdatum der Recherche | Prüfer |
|---|---|---|
| München | 18. Januar 2016 | Witte, Thomas |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

..................................................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 15 18 4462

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

18-01-2016

| Im Recherchenbericht angeführtes Patentdokument | Datum der Veröffentlichung | Mitglied(er) der Patentfamilie | | Datum der Veröffentlichung |
|---|---|---|---|---|
| US 2010277727 A1 | 04-11-2010 | AT | 545014 T | 15-02-2012 |
| | | CA | 2701425 A1 | 30-10-2010 |
| | | CN | 101943656 A | 12-01-2011 |
| | | EP | 2246692 A1 | 03-11-2010 |
| | | JP | 5683836 B2 | 11-03-2015 |
| | | JP | 2010261944 A | 18-11-2010 |
| | | US | 2010277727 A1 | 04-11-2010 |
| US 2008297769 A1 | 04-12-2008 | US | 2008297769 A1 | 04-12-2008 |
| | | WO | 2008151159 A2 | 11-12-2008 |
| WO 2012151358 A2 | 08-11-2012 | CN | 103608661 A | 26-02-2014 |
| | | EP | 2681534 A2 | 08-01-2014 |
| | | JP | 2014517275 A | 17-07-2014 |
| | | US | 2014203173 A1 | 24-07-2014 |
| | | WO | 2012151358 A2 | 08-11-2012 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2012151358 A2